# EUROPEAN PATENT SPECIFICATION

(11) **EP 0 434 724 B1**
(45) Date of publication and mention of the grant of the patent: **18.05.1994**
(21) Application number: 89910225.5
(22) Date of filing: 19.09.1989
(51) Int. Cl.: A61M 16/06, A61M 16/04

(54) **NOZZLE**
DÜSE
EMBOUT

(30) Priority: 19.09.1988 SE 8803304
(43) Date of publication of application: 03.07.1991
(73) Proprietor: FÖRENINGEN LIM, S-214 01 Malmö (SE)
(72) Inventor: BRANTBY, Rolf, S-216 14 Malmö (SE)
(74) Representative: Wagner, Karl Heinz
(86) International application number: SE8900500
(87) International publication number: WO9003199

(56) References cited:
- US-A- 3 107 667
- US-A- 3 139 088
- US-A- 3 768 465
- US-A- 4 495 945

## Description

The present invention relates to a nozzle comprising a base member which is insertable into the mouth of a user and which is provided with individual teeth impressions, whereby the nozzle includes a sealing shield for sealingly engaging the lips and surrounding face portions of the user.

A nozzle of the abovementioned type is substantially previously known from US-A-3 107 667. This nozzle is provided with teeth impressions but it is designed to be used by divers during diving and thus, when the user can actively keep his mouth closed and his teeth and lips pressed against the base member. Another nozzle of the abovementioned type and corresponding to the preamble of claim 1 is substantially known from US-A-3 139 088. This nozzle is not provided with teeth impressions but with a movable sealing shield and it is also designed to be used by persons who actively can keep their mouth closed and their teeth and lips pressed against the base member.

The object of the present invention is to provide a nozzle which is preventing leakage of air from a respirator when the user is relaxing during sleep. This is arrived at according to the invention while the nozzle has obtained the characterizing features of the following claim.

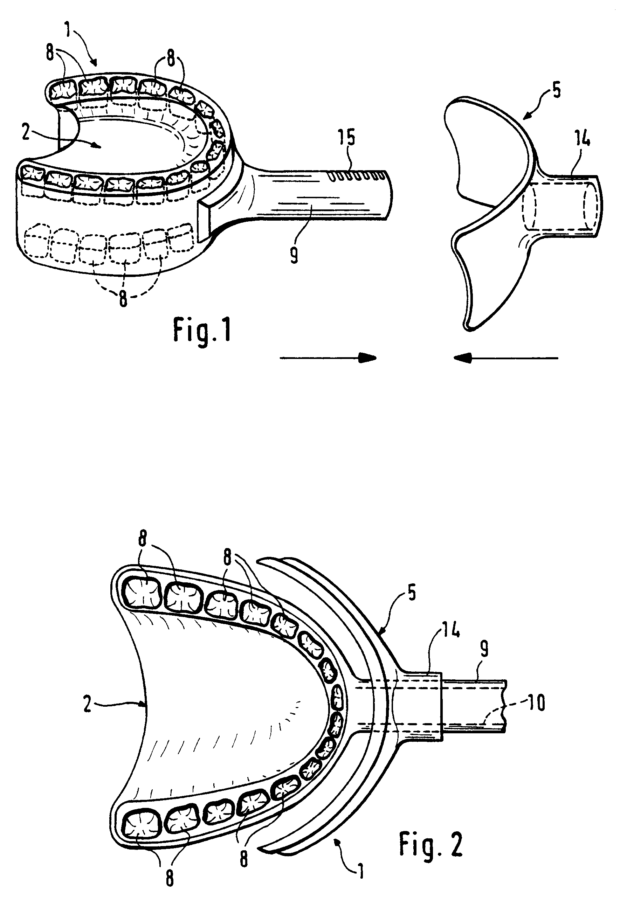

The above and remaining characterizing features will be further described below with reference to the accompanying drawings, in which
fig. 1 is a perspective view of an embodiment of separated base member and shield forming part of a nozzle according to the invention;
fig. 2 is a plan view of base member and shield when connected together; and
fig. 3 is a section through the nozzle in operating position.

The nozzle with these features sees to that the risk of air leakage, i.e. the risk of so called dead-space, is eliminated without the necessity of surgical operations in the patient's throat.

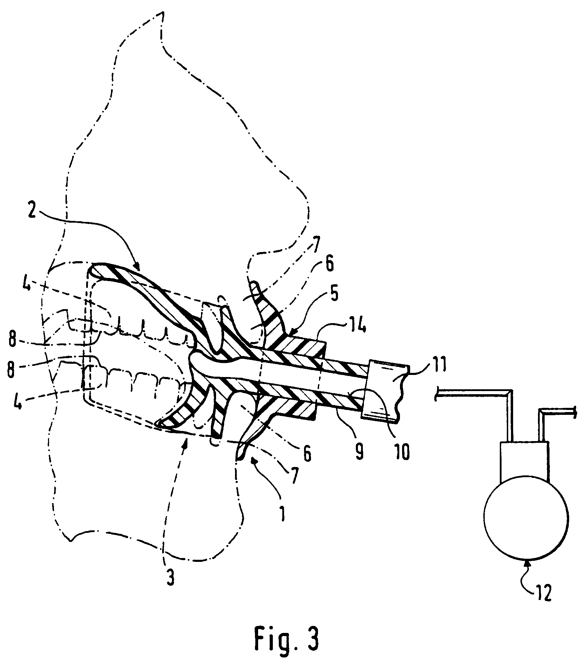

The nozzle illustrated in the drawings is adapted to be taken in the mouth and connected to a respirator. The nozzle is adapted to be used by patients which during the nights, while asleep, tend to get a deficit of oxygen (PO₂-deficit) in the blood or excess of oxygen (PO₂-excess) because of e.g. bad lungs, wrong or blocked signals from the brain or other reasons giving wrong blood gases. For connection of the respirator and for allowing air passage between the respirator and the lungs of the patients, the nozzle has an opening or coupling of any suitable construction. Especially from fig. 3 it is apparent that the nozzle 1 also comprises a base member 2 which is adapted to be disposed in the mouth 3 between the teeth 4 of the patient, and a shield 5 which is adapted to sealingly engage the lips 6 and surrounding portions 7 of the face of the patient from the outside and prevent leakage of air beside the shield.

The base member 2 of the nozzle 1 is individually adapted to the patient's teeth 4 in the upper as well as lower jaw for obtaining a locking of the teeth in the base member. Thus, the base member 2 prevents opening of the patient's mouth 3 during sleep when the muscles of the patient relax, to such an extent that the shield 5 looses its sealing function. Individual adaptation of the base member 2 to the patient's teeth 4 is accomplished by providing the base member with exact impressions 8 of the teeth. These impressions 8 in the base member are obtained in a manner already known and are therefore not further described. It is essential however, that the impressions 8 are designed such that the patient must bite to move the teeth into the impressions and that the base member can be removed from the teeth only by overcoming a certain resistance when removing said base member by hand. Thus, the impressions 8 in the base member 2 must be adapted to the patient's teeth 4 such that the base member is secured in position when the patient is asleep. By manufacturing the base member 2 of a special rubber or plastic material with a dull surface, the achievement of the object is facilitated.

In the embodiment illustrated in the drawings, the base member 2 is individually adapted to all teeth 4 in the patient's upper and lower jaws, but of course, other embodiments are also possible, e.g. a base member only for the front teeth and molars in the upper and lower jaws.

According to the present embodiment of the invention, the shield 5 and base member 2 are provided as two separate members which are connectable to each other. These may be locked by means of a sleeve or agglutinated. Hereby, the shield 5 is disposable at various distances from the tooth impressions 8 in the base member 2 for individual adaptation of the position of the shield in relation to shape and/or thickness of the patient's lips 6 and surrounding face portions 7. Thus, the shield 5 may be positioned with sufficient sealing against the lips 6 and surrounding face portions 7 without e.g. squeezing the lips between the shield 5 and base member 2. In the embodiment shown the shield 5 is threadable onto a tube 9 protruding from the base member 2 between the impressions 8 for the teeth in the upper and lower jaws, said tube 9 defining the opening 10 for permitting air passage between a respirator and the patient's lungs and to which a hose 11 from the respirator 12 is connected. The respirator 12 is also connected to e.g. the electric mains via a conduit 13. In order to facilitate the threading of the shield 5 onto the tube 9 on the base member 2 as well as its setting in correct operating position, the shield also has a tubular portion 14 which is thread onto the tube 9. For permitting fixation of the shield 5 at various locations along the tube 9, said tube 9 and the tubular portion 14 have suitable lock means for this purpose, e.g. a corresponding toothing 15 on each member.

In use, the nozzle 1 is applied such that the base member 2 is put into the mouth 3 of the patient, whereafter the patient bites in said base member such that the teeth 4 are pressed down into the impressions 8 in the base member. The teeth can now be withdrawn from the base member 2 if the patient generates a certain force for this purpose. Thereafter, the shield 5 is thread onto the tube 9 on the base member 2 and moved therealong until it sealingly engage the patient's lips 6 and surrounding face portions 7. In this position, the shield 5 is kept in position by means of the tubular portion 14 and the toothing 15 thereon and on the tube 9. The hose 11 to the respirator 12 is fastened to the tube 9 and the respirator is switched on.

It is obvious for a skilled person that the present invention can be modified within the scope of the following claims without departing from the idea and purpose of the invention. Thus, the base member and the shield to the nozzle can be manufactured in a suitable material and given a design and size suitable for the purpose. Respirator coupling and eventual lock means can also be designed as desired or required for the purpose.

## Claims

1. Nozzle comprising a base member (2) which is insertable into the mouth of a user, the base member being provided with a tube (9) which is connectable to a respirator (12) for supplying air from the respirator into the user's lungs through an opening (10) in the tube (9), a sealing shield (5) for sealingly engaging the lips and surrounding face portions of the user, said sealing shield fixable at various locations along the tube (9) for individual adaption of the sealing shield in relation to the user's lips (6) and surrounding face portions (7) to sealingly engage the lips and surrounding face portions without squeezing the lips and surrounding face portions when air is supplied from a respirator to the lungs characterized in that said nozzle is provided with such individual teeth impressions (8) that the user's teeth (4) are retained in said impressions for preventing opening of the user's mouth (3) when the user is asleep and the muscles of the user relax so that the shield is kept in sealingly contact with the user's lips and surrounding face portions.

## Patentansprüche

1. Düse mit einem in den Mund eines Anwenders einzuführenden Hauptbestandteil (2), der ein mit einem Respirator (12) verbindbares Rohr (9) aufweist, das durch eine Öffnung (10) im Rohr (9) für die Zufuhr von Luft vom Respirator in die Lungen des Anwenders sorgt, und mit einem gegen die Lippen und die umgebenden Gesichtsteile des Anwenders dichtend sich anschmiegenden Dichtungsschild (5), welcher an verschiedenen Stellen entlang das Rohr (9) feststellbar ist zur individuellen Anpassung des Dichtungsschilds an die Lippen (6) und umgebenden Gesichtsteile (7) des Anwenders, so dass der Schild die Lippen und die umgebenden Gesichtsteile dichtend umgibt ohne die Lippen und die umgebenden Gesichtsteile zu klemmen, wenn die Luft vom Respirator den Lungen zugeführt wird, **dadurch gekennzeichnet,** dass die Düse mit solchen individuellen Zahneindrücken (8) versehen ist, dass die Zähne (4) des Anwenders in diesen Eindrücken festgehalten werden, damit der Anwender beim Schlafen und bei entspannten Muskeln an einem Öffnen des Mundes (3) gehindert wird, wodurch der Schild in dichtender Anlage gegen die Lippen und die umgebenden Gesichtsteile gehalten wird.

## Revendications

1. Embout comportant une partie principale (2) destinée à être insérée dans la bouche de l'utilisateur de l'embout et munie d'un tuyau (9) pouvant être relié a un respirateur (12) pour assurer, à travers un orifice (10) du tuyau (9), l'alimentation des poumons de l'utilisateur en air provenant du respirateur, et un écran d'étoupage (5) susceptible de s'ajuster de manière étanche aux lèvres et aux parties voisines du visage de l'utilisateur de l'embout, ledit écran d'étoupage pouvant être fixé en différents endroits le long du tuyau (9) pour permettre l'adaptation individuelle de l'écran aux lèvres (6) et aux parties voisines (7) du visage de l'utilisateur de l'embout de façon qu'il s'ajuste de manière étanche aux lèvres et aux parties voisines du visage de l'utilisateur lorsque l'air est amené du respirateur aux poumons de l'utilisateur, **caractérisé** par le fait que l'embout est muni d'empreintes de dent individuelles (8) telles que les dents (4) de l'utilisateur soient retenues dans lesdites empreintes pour empêcher que l'utilisateur n'ouvre sa bouche (3) lorsqu'il est endormi et ses muscles sont relâchés, afin que l'écran soit maintenu en contact d'étoupage avec les lèvres et les parties voisines du visage de l'utilisateur.
